(19) Europäisches Patentamt
European Patent Office
Office européen des brevets

(11) Numéro de publication: **0 373 072 B1**

# (12) FASCICULE DE BREVET EUROPEEN

(45) Date de publication de fascicule du brevet: **26.10.94**

(51) Int. Cl.$^5$: **C07H 15/12**, C07H 15/203, C07H 17/08, A61K 31/70

(21) Numéro de dépôt: **89403397.6**

(22) Date de dépôt: **07.12.89**

## (54) Ethers et/ou esters lipophiles de D-désosamine, leur procédé de préparation et leur utilisation en tant qu'agents anti-bactériens et antifongiques.

(30) Priorité: **07.12.88 FR 8816067**

(43) Date de publication de la demande: **13.06.90 Bulletin 90/24**

(45) Mention de la délivrance du brevet: **26.10.94 Bulletin 94/43**

(84) Etats contractants désignés: **AT BE CH DE ES GB GR IT LI NL SE**

(56) Documents cités:
**GB-A- 2 175 303**
**US-A- 2 862 921**
**US-A- 3 290 284**

**ANTIMICROBIAL AGENTS AND CHEMOTHERAPY, vol. 28, no. 1, novembre 1985, pages630-633, American Society for Microbiology; I.O. KIBWAGE et al.: "Antibacterialactivities of erythromycins A,B,C, and D and some of their derivatives"**

**CHEMICAL ABSTRACTS, vol. 103, no. 7, 19 août 1985, page 16, résumé no. 47853r,Columbus, Ohio, US; E. SARTORI et al.: "Some erythromycin derivatives arestrong inducers in rats of a cytochrome P-450 very similar to that induced by16 alpha-pregnenolone carbonitrile", &BIOCHEM. BIOPHYS. RES. COMMUN. 1985, 128(3), 1434-9**

(73) Titulaire: **L'OREAL**
**14, Rue Royale**
**F-75008 Paris (FR)**

(72) Inventeur: **Philippe, Michel**
**3, Rue de l'Aubépine**
**F-92160 Antony (FR)**
Inventeur: **Sebag, Henri**
**26, Rue Erlanger**
**F-75016 Paris (FR)**

(74) Mandataire: **Stalla-Bourdillon, Bernard et al**
**Cabinet Nony**
**29, rue Cambacérès**
**F-75008 Paris (FR)**

EP 0 373 072 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet de nouveaux éthers et/ou esters lipophiles de D-désosamine, leur procédé de préparation et leur utilisation en tant qu'agents anti-bactériens et antifongiques.

Ces nouveaux éthers et/ou esters de D-désosamine trouvent tout particulièrement une application dans des compositions pharmaceutiques ou cosmétiques destinées notamment à combattre une prolifération bactérienne qu'elle soit d'origine infectieuse ou non. Selon un article intitulé "Antibacterial Activities of Erythromycins A, B, C and D and some of their Derivatives" par

I. O. KIBWAGE et al. paru dans le journal Antimicrobial Agents and Chemotherapy, Nov. 1985, p.630-633, les erythromycines A, B, C et D présentent une activité anti-bactérienne et ce prinicpalement à l'égard des bactéries gram positives. Ce document indique en outre que la concentration inhibitrice minimum de certains dérivés est supérieure à celle de l'erythromycine A.

Le brevet US 3.290.284 décrit des glycosides de désosamine présentant, entre autres activités, une activité bactéricide.

Les éthers et/ou esters de D-désosamine selon la présente invention se sont révélés, de façon tout à fait surprenante, être pour certains d'entre-eux de puissants agents anti-bactériens alors que les mêmes éthers et/ou esters d'autres hydrates de carbone tels que le glucose ou le cladinose se sont avérés être dépourvus d'activité anti-bactérienne.

La présente invention a pour objet à titre de produits industriels nouveaux des éthers et/ou esters lipophiles, en position 1 et/ou 2, de la D-désosamine, ceux-ci pouvant être représentés par la formule générale suivante:

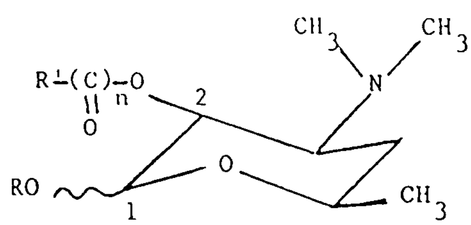

(I)

dans laquelle:

n représente 0 ou 1,

ou R représente la partie aglyconique de l'érythralosamine et R′ représente dans ce cas un radical alkyle, linéaire ou ramifié ayant de 9 à 30 atomes de carbone ou un radical alkényle ayant de 11 à 21 atomes de carbone,

et les anomères $\alpha$ et $\beta$ et leurs mélanges ainsi que les sels des composés de formule (I).

Parmi les radicaux alkyles, linéaires ou ramifiés, ayant de 1 à 30 atomes de carbone, on peut notamment mentionner les radicaux méthyle, éthyle, propyle, isopropyle, butyle, tert-butyle, octyle, nonyle, décyle, undécyle, dodécyle, tétradécyle, hexadécyle, octadécyle et décyl-2 tétradécyle.

Parmi les radicaux alkényles ayant de 5 à 21 atomes de carbone, on peut notamment mentionner les radicaux undécène-10-yle, heptadécaène-8-yle, heptadécadiène-8,11-yle et heptadécatriène-8,11,14-yle.

Le radical cycloalkyle est de préférence un radical cyclopentyle, cyclohexyle ou adamantyle.

Parmi le ou les substituants du radical phényle on peut citer un atome d'halogène tel que le chlore ou le brome, une fonction OH, un radical trifluorométhyle ou un radical alkyle inférieur ayant de 1 à 6 atomes de carbone.

Le radical arylalkyle est de préférence le radical benzyle ou phénéthyle, éventuellement substitué par les mêmes substituants tels que ceux mentionnés ci-dessus pour le radical phényle.

L'aglycone de l'érythralosamine peut être représenté par la formule suivante:

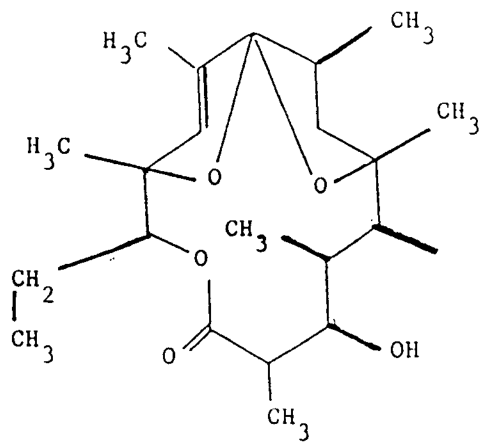

Parmi les éthers et/ou esters de D-désosamine de formule (I) ci-dessus, on peut notamment citer les suivants:

R et R', identiques ou différents, représentent un radical alkyle, linéaire ou ramifié, ayant de 1 à 30 atomes de carbone, un radical alkényle ayant de 5 à 21 atomes de carbone, un radical cycloalkyle ayant de 4 à 10 atomes de carbone, un radical cycloalkylalkyle ayant de 5 à 11 atomes de carbone ou un radical phényle ou arylalkyle éventuellement substitué(s), R pouvant en outre représenter un atome d'hydrogène quand n = 1 et R' pouvant représenter un atome d'hydrogène quand n = 0, le nombre des atomes de carbone de R, R' ou R + R' devant être compris entre 9 et 33 inclus,

<u>Composés n°</u>

1. O-décanoyl-2-O-butyl-1-α,β-D-désosamine,
2. O-dodécanoyl-2-O-butyl-1-α,β-D-désosamine,
3. O-dodécanoyl-2-O-butyl-1-β-D-désosamine,
4. O-dodécanoyl-2-O-méthyl-1-α-β-D-désosamine,
5. O-dodécanoyl-2-O-méthyl-1-α-D-désosamine,
6. O-linoléoyl-2-O-butyl-1-α,β-D-désosamine,
7. O-linoléoyl-2-O-butyl-1-α-D-désosamine,
8. O-octadécyl-2-O-butyl-1-α,β-D-désosamine,
9. O-octadécyl-2-O-butyl-1-α-D-désosamine,
10. O-octadécyl-2-O-butyl-1-β-D-désosamine,
11. O-undécyl-2-O-décyl-1-α,β-D-désosamine,
12. O-undécyl-2-O-décyl-1-α-D-désosamine,
13. O-undécyl-2-O-décyl-1-β-D-désosamine,
14. di-O-octyl-1,2-α,β-D-désosamine,
15. di-O-octyl-1,2-α-D-désosamine,
16. di-O-octyl-1,2-β-D-désosamine,
17. O-(décyl-2)-tétradécyl-2-O-butyl-1-α,β-D-désosamine
18. O-dodécanoyl-2′-érythralosamine,
19. O-oléoyl-2-α,β-D-désosamine,
20. O-décyl-1-α,β-D-désosamine,
21. O-décyl-1-α-D-désosamine,
22. O-décyl-1-β-D-désosamine,
23. O-dodécyl-1-α,β-D-désosamine,
24. O-hexadécyl-1-α,β-D-désosamine,
25. O-(p-nonyl)phényl-1-α,β-D-désosamine.

Selon une forme de réalisation particulièrement préférée, les composés de formule (I) tels que définis ci-dessus, sont des esters-éthers respectivement en position 2 et en position 1 ou des diéthers dont la somme des atomes de carbone de R + R′ est comprise entre 12 et 30 inclus. Parmi les composés ci-dessus énumérés, les composés n°1 à 17 répondent à cette définition.

La présente invention a également pour objet le procédé de préparation des éthers et/ou esters de D-désosamine tel que définis ci-dessus.

En ce qui concerne la préparation des esters en position 2, différents procédés d'estérification peuvent être utilisés mais de préférence celle-ci est réalisée en milieu solvant organique anhydre comme le tétrahydrofuranne, seul ou en mélange avec un autre solvant organique tel que la pyridine ou le N,N-diméthylformamide, en faisant réagir un excès d'anhydride mixte de l'acide choisi, préparé in situ, par exemple à partir de chloroformiate d'éthyle et de l'acide choisi, sur la D-désosamine ou sur un monoéther en position 1 de la D-désosamine.

D'autres méthodes d'estérification peuvent être employées, notamment la méthode utilisant les imidazolides des acides choisis dans un solvant anhydre comme la pyridine ou le N,N-diméthylformamide en présence d'une base comme le tert-butanolate de potassium ou l'imidazolidure de sodium, mais ces méthodes donnent de manière générale des rendements plus faibles.

Les réactions d'éthérification sont réalisées, pour la position en 1, sur la D-désosamine, en présence de l'alcool choisi (ROH) et d'un acide minéral tel que l'acide sulfurique ou chlorhydrique ou d'un acide organique tel que l'acide paratoluène sulfonique, éventuellement dans un solvant organique tel que le N,N-diméthylformamide à une température d'environ 80 ° C.

Les éthers en position 2 sont de préférence obtenus en faisant réagir un halogénure R′X, (X étant Cl ou Br) ou un dérivé tosylé sur un monoéther en 1 de la D-désosamine, en présence d'une base comme le tert-butanolate de potassium, dans un solvant organique anhydre tel que le dioxane ou le N,N-diméthylformamide à environ 80 ° C.

La présente invention a également pour objet l'utilisation des éthers et/ou esters de D-désosamine tels que définis ci-dessus comme agent antibactérien ou antifongique et notamment en thérapeutique humaine et vétérinaire et en cosmétique.

Les compositions pharmaceutiques peuvent être administrées par voie topique, orale, parentérale ou rectale et sont destinées plus particulièrement au traitement d'affections d'origine bactérienne et les compositions cosmétiques sont plus particulièrement destinées au traitement de diverses dermatoses notamment de l'acné. Ces compositions qu'elles soient destinées à un traitement thérapeutique ou cosmétique peuvent se présenter sous diverses formes en particulier sous forme anhydre et contiennent au moins un éther et/ou ester de D-désosamine tel que défini ci-dessus à une concentration comprise entre 0,001 et 10% mais de préférence entre 0,05 et 3% en poids par rapport au poids total de la composition.

Pour la préparation de compositions selon l'invention contenant en tant que substance active au moins un éther et/ou ester de D-désosamine, on peut faire appel à divers véhicules et adjuvants décrits dans la littérature pour la pharmacie, la cosmétique et les domaines apparentés.

Pour la préparation des solutions, on peut utiliser par exemple un (ou des) solvant(s) organique(s) acceptables(s) d'un point de vue physiologique.

Les solvants organiques acceptables sont pris notamment dans le groupe constitué par l'acétone, l'alcool isopropylique, les triglycérides d'acide gras, les éthers de glycol, les esters d'alkyle en $C_1$-$C_4$ d'acides à courte chaîne, les alcoyl-éthers du polytétrahydrofuranne ainsi que les silicones volatiles tels que les cyclométhicones.

Les compositions selon l'invention peuvent également renfermer des épaississants tels que la cellulose et/ou des dérivés de la cellulose à raison de 0,5 à 20% en poids par rapport au poids total de la composition.

Les compositions selon l'invention peuvent en outre contenir en association, avec au moins un éther et/ou ester de D-désosamine tel que défini ci-dessus, au moins un autre agent anti-bactérien ou anti-acnéique connu.

On peut, si nécessaire, ajouter un adjuvant usuel pris dans le groupe formé par les agents anti-oxydants, les agents conservateurs, les parfums et les colorants.

Parmi les anti-oxydants utilisables, on citera par exemple la tert-butylhydroxyquinone, le butylhydroxyanisole, le butylhydroxytoluène et l'$\alpha$-tocophérol et ses dérivés.

Les transformations pharmacologiques et galéniques des composés selon l'invention s'effectuent de façon connue.

Les formes galéniques peuvent être pour la voie topique, des crèmes, des laits, des gels, des lotions plus ou moins épaissies, des lotions portées par des tampons, des timbres, des pommades, des sticks ou bien des formulations aérosols se présentant sous forme de sprays ou de mousses.

Les compositions par voie orale peuvent se présenter sous forme de comprimés, de gellules, de dragées, de sirops, de suspensions, d'émulsions, de poudres, de granulés ou de solutions.

Les compositions peuvent également se présenter sous forme de suppositoires.

Le traitement anti-bactérien et/ou anti-fongique à l'aide des compositions topiques selon l'invention consiste à appliquer deux ou trois fois par jour une quantité suffisante, sur les zones de la peau à traiter et ceci pendant une période de temps de 6 à 30 semaines et de préférence de 12 à 24 semaines.

Les compositions selon l'invention peuvent également être utilisées à titre préventif, c'est-à-dire sur les zones de peau susceptibles d'être atteintes d'une prolifération bactérienne.

Bien qu'il ait été fait plus particulièrement référence à une utilisation des éthers et/ou esters de D-désosamine comme composés actifs dans des compositions pharmaceutiques et cosmétiques, ceux-ci peuvent également être utilisés en tant que conservateurs pour de telles compositions et trouver par ailleurs diverses applications dans d'autres domaines de l'industrie tels que l'agriculture, la papeterie, les peintures et vernis, le traitement des eaux, etc...

ETUDE COMPARATIVE SUR L'ACTIVITE DES ESTERS ET/OU ETHERS LIPOPHILES DE D-DESOSAMINE

L'activité des esters et/ou éthers de D-désosamine a été étudiée par la méthode de dilution en vue de déterminer la concentration minimale inhibitrice (CMI), méthode décrite et employée par G. A. DENYS et al, Antimicrobial Agents and Chemotherapy (1983) 23, 335-337 et J.J. LEYDEN et al, J. Am. Acad. Dermatol. (1983) 8 (1) 41-5, en utilisant comme souche de propionibacterium acnes, la souche P37 fournie par CUNLIFFE et HOLLAND.

Cette souche P37 a fait l'objet des études décrites dans les publications suivantes:

- J. GREENMAN, K.T. HOLLAND et W.J. CUNLIFFE, Journal of General Microbiology (1983) 129, 1301-1307,
- E. INGHAM, K.T. HOLLAND, G. GOWLAND et W.J. CUNLIFFE, ibid (1980) 118, 59-65 et
- K.T. HOLLAND, J. GREENMAN et W.J. CUNLIFFE, Journal of Applied bacteriology (1979) 47, 383-394.

Sélection et isolement des populations sensibles et résistantes.

La souche P37 est sensible à l'érythromycine comme le montre la concentration minimale inhibitrice (CMI = 0,78µg/ml).

En revanche, après 8 sous-cultures successives dans le même milieu, (RCM*19/20, DMSO 19/20 en volume) en vue d'obtenir une stabilisation progressive de cette souche à ce milieu, une résistance progressive à l'érythromycine se manifeste sous la forme suivante:

- après étalage d'un inoculum standardisé (DO = 1,8 à 450nm) sur milieu gélosé (RCM + furazolidone), en boîte de Petri, un disque de 9mm de diamètre est déposé au centre de celle-ci. Sur le disque, 50µg d'érythromycine (en solution dans le DMSO) sont déposés.
- après 6 jours à 36 °C en milieu anaérobie (système GAS-PAK, B.B.L) une zone d'inhibition de la pousse de la souche est nettement visible (diamètre total = 42mm), la majorité des colonies étant située à la périphérie de la zone d'inhibition.

En revanche à l'intérieur de celle-ci quelques colonies apparaissent nettement.

Les deux types de colonies sont alors prélevés par arrachage du milieu gélosé (Anse de platine stérilisé):

1) à l'intérieur de la zone d'inhibition on prélève les souches dénommées P37 $E^{\ominus}$ en raison de leur résistance apparente à l'érythromycine.

2) à 1cm au-delà de la périphérie de la zone d'inhibition, on prélève les souches dénommées P37 $E^{\oplus}$.

Après isolement et culture, les souches P37 $E^{\oplus}$ et P37 $E^{\ominus}$ montrent effectivement des sensibilités très différentes à l'érythromycine illustrées par les valeurs suivantes des CMI respectives.

| | CMI (µg/ml) |
|---|---|
| P37 | 0,78 |
| P37 $E^{\oplus}$ | 0,78 |
| P37 $E^{\ominus}$ | 50 |

* Reinforced Clostridium Medium (OXOID).

Ce phénomène est confirmé par l'étude de la CI 50 (concentration inhibitrice à 50%) qui représente la concentration d'érythromycine où, à un temps constant de culture, 50% de survivants parmi la population sont retrouvés.

| | CI 50 (µg/ml) |
|---|---|
| P37 | 50 |
| P37 E$^{\oplus}$ | 5 |
| P37 E$^{\ominus}$ | 100 |

La concentration minimale inhibitrice (CMI) exprimée en µg/ml des esters et/ou éthers de D-désosamine testés vis-à-vis des souches de propionibactérium Acnes P37, P37 E$^{\oplus}$ et P37 E$^{\ominus}$ et de staphilococcus epidermidis ATCC 12228 est reportée dans le tableau I suivant:

TABLEAU I

| Ethers et/ou esters de D-désosamine Composés N° (voir page 3) | ATCC 12228 | P37 | P37 E$^{\oplus}$ (sensible) | P37 E$^{\ominus}$ (résistante) |
|---|---|---|---|---|
| 1 | 5 | 9 | 9 | 3,2 |
| 2 | 3,5 | 0,4 | 0,6 | 0,9 |
| 3 | - | 15 | 0,1 | 1,9 |
| 4 | 3,8 | 25 | 15 | 15 |
| 5 | 3,2 | 50 | 50 | 50 |
| 6 | 0,2 | 0,4 | 0,7 | 0,8 |
| 7 | 1,1 | 0,15 | 1,1 | 1,1 |
| 8 | 2,2 | 0,25 | <0,03 | 8,8 |
| 9 | 0,8 | 0,1 | 27,5 | 27,5 |
| 10 | 0,04 | 3,12 | 0,09 | 1,56 |
| 11 | 0,7 | 4 | 0,7 | 0,8 |
| 12 | 0,8 | 1,8 | 1 | 0,8 |
| 13 | 0,06 | 1 | 0,5 | 0,02 |
| 14 | 0,5 | 0,5 | 0,6 | 0,1 |
| 15 | 0,3 | 1,8 | 1 | 0,4 |
| 16 | 1,2 | 0,7 | 0,3 | 0,15 |
| 17 | - | 12,5 | 11 | 6 |
| 18 | 1,6 | 0,4 | 12,5 | 0,4 |

EXEMPLES DE PREPARATION

EXEMPLE 1

Préparation du O-décanoyl-2-O-butyl-1-α,β-D-désosamine

Dans un ballon, sous atmosphère inerte, on dissout 2,8g (16,3mmoles) d'acide décanoïque dans 50ml de tétrahydrofuranne anhydre; le mélange réactionnel est refroidi à 0°C puis on verse 3ml de pyridine anhydre et 1,6ml (16,6mmoles) de chloroformiate d'éthyle. La solution est agitée 5 minutes et on ajoute 2,5g d'hydrogénocarbonate de sodium puis 1,5g (6,5mmoles) de O-butyl-α,β-D-désosamine préalablement dissous dans 70ml de tétrahydrofuranne. Le mélange réactionnel est alors laissé sous agitation pendant 10 heures en laissant remonter à température ambiante (chromatographie sur couche mince de gel de silice: chlorure de méthylène/méthanol 10%). La solution est versée sur 60ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C) en utilisant l'éluant: acétate d'éthyle (4)/hexane (6) pour aboutir à l'isolement de 1,8g (72% de rendement) de O-décanoyl-2-O-butyl-1-α,β-D-désosamine.

| Microanalyse: $C_{22}$ $H_{43}$ $NO_4$, $1H_2O$; M = 394,6 | | |
|---|---|---|
| | C | H |
| Calc % :<br>Tr % : | 66,96<br>67,49 | 11,24<br>11,20 |

La R.M.N. du $^{13}C$ ($CDCl_3$, réf. interne T.M.S.) confirme l'estérification des anomères $\alpha$ et $\beta$ en position 2 du cycle désosamine.

EXEMPLE 2

Préparation du O-dodécanoyl-2-O-butyl-1-$\alpha$,$\beta$-D-désosamine

Dans un ballon, sous atmosphère inerte, on dissout 3,35g (16,7mmoles) d'acide dodécanoïque dans 45ml de tétrahydrofuranne anhydre; le mélange réactionnel est refroidi à 0°C puis on verse 3ml (38mmoles) de pyridine anhydre et 1,6ml (16,6mmoles) de chloroformiate d'éthyle. La solution est agitée 5 minutes et on ajoute 2,5g (30mmoles) d'hydrogé- nocarbonate de sodium puis 1,5g (6,5mmoles) de O-butyl-$\alpha$,$\beta$-D-désosamine préalablement dissous dans 100ml de tétrahydrofuranne. Le mélange réactionnel est alors laissé sous agitation pendant 10 heures en laissant remonter à température ambiante (chromatographie sur couche mince de gel de silice: chlorure de méthylène/méthanol 10%). La solution est versée sur 60ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C.) en utilisant l'éluant: acétate d'éthyle (4)/hexane (6) pour aboutir à l'isolement de 2,2g (82% de rendement) de O-dodécanoyl-2-O-butyl-1-$\alpha$,$\beta$-D-désosamine.

| Microanalyse: $C_{24}$ $H_{47}$ $NO_4$ ; M = 413,65 | | | |
|---|---|---|---|
| | C | H | N |
| Calc % :<br>Tr % : | 69,69<br>69,59 | 11,45<br>11,40 | 3,39<br>3,24 |

R.M.N. du $^{13}C$ ($CDCl_3$, réf. interne T.M.S.)

Les effets $\gamma$ négatifs en 1 (-3,3ppm et -2,5ppm respectivement pour les anomères $\alpha$ et $\beta$ ) ainsi qu'en 3 (-2,8ppm et -1,8ppm pour les anomères $\alpha$ et $\beta$ ) montrent l'estérification en position 2.

EXEMPLE 3

Préparation du O-dodécanoyl-2-O-butyl-1-$\beta$-D-désosamine

Dans un ballon, sous atmosphère inerte, on dissout 3,35g (16,7mmoles) d'acide dodécanoïque dans 45ml de tétrahydrofuranne anhydre; le mélange réactionnel est refroidi à 0°C puis on verse 3ml de pyridine anhydre et 1,6ml (16,6mmoles) de chloroformiate d'éthyle. La solution est agitée 5 minutes et on ajoute 2,5g d'hydrogénocarbonate de sodium puis 1,5g (6,5mmoles) de O-butyl-$\beta$-D-désosamine préalablement dissous dans 100ml de tétrahydrofuranne. Le mélange réactionnel est alors laissé sous agitation pendant 10 heures en laissant remonter à température ambiante (chromatographie sur couche mince de gel de silice: chlorure de méthylène/méthanol 10%). La solution est versée sur 60ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C) en utilisant l'éluant: acétate d'éthyle (4)/hexane (6) pour aboutir à l'isolement de 2g (75% de rendement) de O-dodécanoyl-2-O-butyl-1-$\beta$-D-désosamine purs.

| Microanalyse: $C_{24}H_{47}NO_4$; M = 413,65 | | | |
|---|---|---|---|
| | C | H | N |
| calc % :<br>Tr % : | 69,69<br>69,04 | 11,45<br>11,89 | 3,39<br>2,96 |

$[\alpha]_D^{20}$ = + 19° (c = 0,5mg/ml, dichlorométhane).
R.M.N. du $^{13}C$ ($CDCl_3$, réf. interne T.M.S.)
Effets $\gamma$ négatifs en 1 (-2,7ppm) et 3 (-2,3ppm) indiquent la position de l'ester en 2.

## EXEMPLE 4

### Préparation du O-dodécanoyl-2-O-méthyl-1-α,β-D-désosamine

Ce composé est préparé selon le même mode opératoire que celui décrit à l'exemple 1 à partir du O-méthyl-1-α,β-D-désosamine (rendement 68%).

| Microanalyse: $C_{21}H_{41}NO_4$ ; M = 371,6. | | |
|---|---|---|
| | C | H |
| Calc %:<br>Tr% : | 67,88<br>67,65 | 11,12<br>11,08 |

La R.M.N. du $^1H$ ($CDCl_3$, réf. int. T.M.S.) confirme la structure attendue.

## EXEMPLE 5

### Préparation du O-dodécanoyl-2-O-méthyl-1-α-D-désosamine

Ce composé est préparé selon le même mode opératoire que celui décrit à l'exemple 1 à partir du O-méthyl-1-α,β-D-désosamine et purification du mélange anomérique (rendement 45%).

| Microanalyse: $C_{21}H_{41}NO_4$ ; M = 371,6. | | |
|---|---|---|
| | C | H |
| Calc%:<br>Tr%: | 67,88<br>67,99 | 11,12<br>11,18 |

La R.M.N. du $^1H$ ($CDCl_3$, réf. Int. T.M.S.) confirme la structure attendue.

## EXEMPLE 6

### Préparation du O-linoléoyl-2-O-butyl-1-α,β-D-désosamine

Ce composé est préparé selon le même mode opératoire que celui décrit à l'exemple 1 à partir du O-butyl-1-α,β-D-désosamine (rendement 70%).

| Microanalyse: $C_{30}H_{55}NO_4$ , $0,5H_2O$; M = 502,8 | | | |
|---|---|---|---|
| | C | H | N |
| Calc%:<br>Tr%: | 71,66<br>71,91 | 11,22<br>11,06 | 2,78<br>2,77 |

R.M.N. du 1 H ($CDCl_3$ , T.M.S comme réf. int.) confirme la structure attendue.

EXEMPLE 7

Préparation du O-linoléoyl-2-O-butyl-1-α-D-désosamine

Ce composé est préparé selon le même mode opératoire que celui décrit à l'exemple 1 à partir du O-butyl-1-α,β-D-désosamine et purification du mélange anomérique (rendement 45%).
$[\alpha]_D^{20}$ = + 71° (C=0,85mg/ml, dichlorométhane)

| Microanalyse: $C_{30}H_{55}NO_4$ ; M= 493,8 | | | |
|---|---|---|---|
| | C | H | N |
| Calc%:<br>Tr%: | 72,97<br>72,38 | 11,23<br>11,00 | 2,84<br>2,85 |

R.M.N. du $^{13}C$ ($CDCl_3$, T.M.S. en réf. int.): les effets γ négatifs en 1 (-2,3 ppm) et 3 (-1,7 ppm) montrent l'estérification en position 2 et la position anomérique en α.

EXEMPLE 8

Préparation du O-oléoyl-2-α,β-D-désosamine

Ce composé est préparé selon le même mode opératoire que celui décrit à l'exemple 1 à partir de l'α,β-D-désosamine (50% de rendement).

| Microanalyse: $C_{26}H_{49}NO_4$, 0,5$H_2O$ ; M = 448,7. | | | |
|---|---|---|---|
| | C | H | N |
| Calc%:<br>Tr%: | 69,59<br>69,57 | 11,23<br>11,11 | 3,12<br>3,12 |

R.M.N. du $^{13}C$ ($CDCl_3$, réf. int. T.M.S.): les déplacements chimiques des $C_1$ α (90,78 ppm), $C_1$β (96,68 ppm), $C_2$α (70,93 ppm) et $C_2$β (72,78 ppm) montrent l'estérification en position 2.
R.M.N. du $^1H$ ($CDCl_3$, réf. int. T.M.S.): les déplacements chimiques et couplages des $H_1$ ($H_1$ de l'anomère β : δ : 4,46 ppm -J= 7,6 Hz; $H_1$ de l'anomère α : δ : 5,21 ppm - J = 3,3 Hz) confirment le mélange anomérique α,β avec estérification en position 2.

EXEMPLE 9

Préparation du O-dodécanoyl-2′ érythralosamine

Ce composé est préparé selon le même mode opératoire que celui décrit à l'exemple 1 à partir de l'érythralosamine (65% de rendement).
$[\alpha]_D^{20}$ = + 0,29° (C= 3mg/ml, dichlorométhane).

| Microanalyse: $C_{41}H_{71}NO_9$ ; M = 722 | | | |
|---|---|---|---|
| | C | H | N |
| Calc%:<br>Tr%: | 68,20<br>67,78 | 9,91<br>9,96 | 1,94<br>1,94 |

R.M.N. du $^{13}C$ confirme la structure attendue ($CDCl_3$, réf. int. T.M.S.).

EXEMPLE 10

Préparation du O-décyl-$_1$-α,β-D-désosamine

Dans un ballon muni d'un réfrigérant, on dissout 3g (17mmoles) de D-désosamine dans 30ml de décanol puis on ajoute 2ml d'acide sulfurique à 95% et on laisse agiter à 70 °C pendant 10 heures (C.C.M.: dichlorométhane/ méthanol 15%); le milieu réactionnel est ensuite extrait (dichlorométhane/ eau bicarbonatée), la phase organique lavée puis séchée sur sulfate de sodium pour donner 4,5g (83% de rendement) de O-décyl-1-α,β -D-désosamine.

| Microanalyse: $C_{18}H_{37}NO_3$ ; M = 315,5 | | | |
|---|---|---|---|
| | C | H | N |
| Calc% : | 68,53 | 11,82 | 4,44 |
| Tr%: | 68,38 | 11,71 | 4,27 |

R.M.N. $^{13}C$ ($CDCl_3$ , réf. int. T.M.S.) : les déplacements chimiques des $C_1α$ (98,53 ppm.) et $C_1β$ (103,2 ppm.) montrent la glycosylation en position 1.

EXEMPLE 11

Préparation du O-décyl-1-α-D-désosamine

Ce composé est isolé par purification H.P.L.C. (dichlorométhane/ méthanol 8%) du mélange anomérique décrit à l'exemple 10.

| Microanalyse : $C_{18}H_{37}NO_3$ ; M = 315,5 | | | |
|---|---|---|---|
| | C | H | N |
| Calc%: | 68,53 | 11,82 | 4,40 |
| Tr%: | 68,20 | 11,46 | 4,16 |

La R.M.N. du $^1H$ ($CDCl_3$ , réf. int. T.M.S.) confirme la structure attendue.

EXEMPLE 12

Préparation du O-décyl-1-β-D-désosamine

Ce composé est isolé par purification H.P.L.C. (dichlorométhane/ méthanol 8%) du mélange anomérique décrit à l'exemple 10.

| Microanalyse: $C_{18}H_{37}NO_3$ , $0,5H_2O$ ; M = 324,5 | | | |
|---|---|---|---|
| | C | H | N |
| Calc%: | 66,62 | 11,82 | 4,32 |
| Tr%: | 66,03 | 11,94 | 4,64 |

La R.M.N. du $^1H$ ($CDCl_3$ , réf. int. T.M.S.) confirme la structure attendue.

EXEMPLE 13

Préparation du O-dodécyl-1-α,β-D-désosamine

Ce composé a été synthétisé selon le mode opératoire décrit à l'exemple 10 à partir de la D-désosamine et du dodécanol (rendement 85%).

| Microanalyse: $C_{20}H_{41}NO_3$ , 0,75 $H_2O$ ; M = 357,1 | | | |
|---|---|---|---|
| | C | H | N |
| Calc%:<br>Tr%: | 67,26<br>67,37 | 11,98<br>12,09 | 3,92<br>3,82 |

La R.M.N. du $^1H$ ($CDCl_3$ , réf. int. T.M.S.) confirme la structure attendue.

EXEMPLE 14

Préparation du O-hexadécyl-1-α,β-D-désosamine

Ce composé a été synthétisé selon le mode opératoire décrit à l'exemple 10 à partir de la D-désosamine et de l'hexadécanol (rendement 85%).

| Microanalyse: $C_{24}H_{49}NO_3$ ; M = 399,7 | | | |
|---|---|---|---|
| | C | H | N |
| Calc%:<br>Tr%: | 72,12<br>71,60 | 12,36<br>12,24 | 3,50<br>3,44 |

R.M.N. du $^{13}C$ ($CDCl_3$, T.M.S. réf. int.) : les déplacements chimiques des $C_1$ β (103,75 ppm.), $C_1$ α - (98,27 ppm.), $C_2$ β (69,71 ppm.) et $C_2$α (68,43 ppm.) sont en accord avec la formule proposée.
La R.M.N. du $^1H$ ($CDCl_3$ , réf. int. T.M.S) confirme cette structure.

EXEMPLE 15

Préparation du O-octadécyl-2-O-butyl-1-α,β-D-désosamine

Dans un ballon muni d'un réfrigérant, sous atmosphère inerte, on dissout 4g (17mmoles) de O-butyl-1-α,β-D-désosamine dans 150ml de N,N-diméthylformamide anhydre et l'on ajoute 3,9g (34mmoles) de tert-butanolate de potassium, l'ensemble est agité à température ambiante pendant 45mn puis on ajoute 11,5g (34mmoles) de bromooctadécane; le milieu réactionnel est alors agité pendant 12 heures à 100°C. Le mélange est ensuite extrait (acétate éthyle/eau), la phase organique lavée et séchée sur sulfate de magnésium. Après évaporation des solvants, le produit brut est chromatographié sur colonne de gel de silice (acétate d'éthyle (5)/heptane (5)) pour conduire à 7 g (83 % de rendement) de O-octadécyl-2-O-butyl-1-α,β-D-désosamine.

| Microanalyse: $C_{30}H_{61}NO_3$ , 0,5$H_2O$ ; M = 492,8. | | | |
|---|---|---|---|
| | C | H | N |
| Calc%:<br>Tr%: | 73,11<br>73,13 | 12,68<br>12,79 | 2,84<br>2,84 |

La R.M.N. du $^1H$ ($CDCl_3$ , réf. int. TMS) confirme la structure attendue.

EXEMPLE 16

Préparation du O-octadécyl-2-O-butyl-1-α-D-désosamine

Ce composé a été isolé par purification H.P.L.C. du mélange anomérique décrit à l'exemple 15 (acétate d'éthyle (5)/heptane (5)).
$[\alpha]_D^{20}$ = + 79° (C=0,6mg/ml, dichlorométhane).

| Microanalyse : $C_{30}H_{61}NO_3$ ; M = 483,8. | | | |
|---|---|---|---|
| | C | H | N |
| Calc%:<br>Tr% : | 74,48<br>73,98 | 12,71<br>12,74 | 2,90<br>2,88 |

La R.M.N. $^1H$ ($CDCl_3$, réf. int. T.M.S.) confirme la structure attendue.

EXEMPLE 17

Préparation du O-octadécyl-2-O-butyl-1-β-D-désosamine

Ce composé a été isolé par purification H.P.L.C. (acétate d'éthyle (5)/heptane (5)) du mélange anomérique décrit à l'exemple 15.

$[\alpha]_D^{20}$ = - 15° (0,4mg/ml, dichlorométhane)

| Microanalyse: $C_{30}H_{61}NO_3$, $0,5H_2O$ ; M = 492,8 | | | |
|---|---|---|---|
| | C | H | N |
| Calc%:<br>Tr%: | 73,11<br>73,70 | 12,68<br>12,74 | 2,84<br>2,79 |

R.M.N. du $^{13}C$ ($CDCl_3$, réf. int. T.M.S.) : les déplacements chimiques du $C_1$ (104,9 ppm) et du $C_3$ - (63,95 ppm) indiquent à la fois, l'anomérisation en β et l'éthérification par le chainon $C_{18}$ en position 2.

EXEMPLE 18

Préparation du O-undécyl-2-O-décyl-1-α,β-D-désosamine

Ce composé a été préparé à partir du O-décyl-1-α,β-D-désosamine et du bromo-1-undécane selon le mode opératoire décrit à l'exemple 15 (rendement 72%).

| Microanalyse : $C_{29}H_{59}NO_3$ ; M = 469,8. | | | |
|---|---|---|---|
| | C | H | N |
| Calc%:<br>Tr%: | 74,14<br>74,07 | 12,66<br>12,62 | 2,98<br>2,89 |

R.M.N. du $^{13}C$ ($CDCl_3$, réf. int. T.M.S.): les déplacements chimiques des $C_1$ α (96,60 ppm.) et $C_1$ β - (105,01 ppm.) montrent l'éthérification en position 2.

EXEMPLE 19

Préparation du O-undécyl-2-O-décyl-1-α-D-désosamine

Ce composé a été isolé par purification H.P.L.C. (acétate d'éthyle (5)/heptane (5)) du mélange anomérique décrit à l'exemple 18.

$[\alpha]_D^{20}$ = + 66° (C = 1,4mg/ml, dichlorométhane).

| Microanalyse : $C_{29}H_{59}NO_3$ ; M = 469,8. | | | |
|---|---|---|---|
| | C | H | N |
| Calc%:<br>Tr%: | 74,14<br>74,04 | 12,66<br>12,68 | 2,98<br>2,97 |

La R.M.N. du $^1H$ ($CDCl_3$ , réf. int. T.M.S.) confirme la structure attendue.

EXEMPLE 20

Préparation du O-undécyl-2-O-décyl-l-β-D-désosamine

Ce composé a été isolé par purification H.P.L.C. (acétate d'éthyle (5)/heptane (5)) du mélange anomérique décrit à l'exemple 18.
$[\alpha]_D^{20}$ = - 18° (C=1,7mg/ml, dichlorométhane).

| Microanalyse: $C_{29}H_{59}NO_3$ ; M = 469,8. | | | |
|---|---|---|---|
| | C | H | N |
| Calc%:<br>Tr%: | 74,14<br>74,25 | 12,66<br>12,55 | 2,98<br>2,72 |

La R.M.N. du $^1H$ ($CDCl_3$, réf. int. T.M.S.) confirme la structure attendue.

EXEMPLE 21

Préparation du di-O-octyl-1,2-α,β-D-désosamine

Ce composé a été préparé à partir de l'octyl-1-α,β-D-désosamine et du bromo-1-octane selon le mode opératoire décrit à l'exemple 15 (rendement 70%).

| Microanalyse: $C_{24}H_{49}NO_3$ ; M = 399,7. | | | |
|---|---|---|---|
| | C | H | N |
| Calc%:<br>Tr%: | 72,13<br>71,62 | 12,36<br>12,51 | 3,50<br>3,44 |

R.M.N. du $^1H$ ($CDCl_3$ , réf. int. T.M.S.) confirme la structure attendue.

EXEMPLE 22

Préparation du di-O-octyl-1,2-α-D-désosamine

Ce composé est isolé par purification H.P.L.C. (acétate d'éthyle (5)/heptane (5)) du mélange anomérique décrit à l'exemple 21.

| Microanalyse: $C_{24}H_{49}NO_3$ ; M = 399,7. | | | |
|---|---|---|---|
| | C | H | N |
| Calc%:<br>Tr%: | 72,13<br>71,88 | 12,36<br>12,33 | 3,50<br>3,52 |

La R.M.N. $^1H$ ($CDCl_3$, réf. int. T.M.S.) confirme la structure attendue.

EXEMPLE 23

Préparation du di-O-octyl-1,2-β-D-désosamine

Ce composé est isolé par purification H.P.L.C. (acétate d'éthyle (5)/heptane (5)) du mélange anomérique décrit dans l'exemple 21.

| Microanalyse : $C_{24}H_{49}NO_3$ ; M = 399,7 | | | |
|---|---|---|---|
| | C | H | N |
| Calc%: | 72,13 | 12,36 | 3,50 |
| Tr%: | 72,12 | 12,35 | 3,55 |

R.M.N. du $^{13}C$ ($CDCL_3$), réf. int. T.M.S.) montre par le déplacement chimique du $C_1$ à 104,98 ppm. que l'anomère est β.

EXEMPLE 24

Préparation du O-(décyl-2)-tétradécyl-2-O-butyl-1-α,β-D-désosamine

Ce composé est préparé à partir du O-décyl-2-O-tosyl-1-tétradécanol et du O-butyl-1-α,β-D-désosamine selon le mode opératoire décrit à l'exemple 15 (rendement 72%).

| Microanalyse: $C_{36}H_{73}NO_3$ ; M = 568 | | | |
|---|---|---|---|
| | C | H | N |
| Calc%: | 76,12 | 12,95 | 2,46 |
| Tr%: | 76,36 | 12,78 | 2,09 |

La R.M.N. du $^{1}H$ ($CDCl_3$ , réf. int. T.M.S.) confirme la structure attendue.

EXEMPLE 25

Préparation du O-(p-nonyl)phényl-1-α,β-D-désosamine

Ce composé est préparé à partir du p-nonylphénol et de la D-désosamine selon le mode opératoire décrit à l'exemple 10 (rendement 60%).

| Microanalyse: $C_{23}H_{39}NO_3$ , $0,5H_2O$; M = 386,6 | | | |
|---|---|---|---|
| | C | H | N |
| Calc%: | 71,45 | 10,42 | 3,62 |
| Tr%: | 71,46 | 10,51 | 3,15 |

La R.M.N. du $^{1}H$ ($CDCl_3$ , réf. int. T.M.S.) confirme la structure attendue.

COMPOSITIONS PHARMACEUTIQUES ET COSMETIQUES

A - GELS ANTISEPTIQUES TOPIQUES

| | | |
|---|---|---|
| 1. | Hydroxypropyl cellulose<br>Butylhydroxytoluène<br>O-linoléoyl-2-O-butyl-1-α,β-D-désosamine<br>Isopropanol q.s.p. | 1g<br>0,05g<br>0,5g<br>100g |
| 2. | Hydroxypropyl cellulose<br>Butylhydroxytoluène<br>O-dodécanoyl-2-O-butyl-1-α,β-D-désosamine<br>Isopropanol q.s.p. | 1,5g<br>0,05g<br>0,3g<br>100g |

B. LOTIONS ANTISEPTIQUES TOPIQUES

| | | |
|---|---|---|
| 1. | Butylhydroxytoluène<br>O-dodécanoyl-2'-érythralosamine<br>Triglycérides d'acides gras en $C_8$-$C_{12}$ q.s.p. | 0,05g<br>1g<br>100g |
| 2. | Butylhydroxytoluène<br>O-octadécyl-2-O-butyl-1-α,β-D-désosamine<br>Triglycérides d'acides gras en $C_8$-$C_{12}$ q.s.p. | 0,05g<br>0,7g<br>100g |
| 3. | Butylhydroxytoluène<br>O-undécyl-2-O-décyl-α,β-D-désosamine<br>Triglycérides d'acides gras en $C_8$-$C_{12}$<br>Isopropanol q.s.p. | 0,05g<br>1g<br>40g<br>100g |
| 4. | Butylhydroxytoluène<br>di-O-octyl-1,2-α,β-D-désosamine<br>Isopropanol q.s.p. | 0,05g<br>0,5g<br>100g |

C. STICK ANTISEPTIQUE

| | |
|---|---|
| Vaseline blanche<br>Huile de vaseline<br>Paraffine raffinée<br>O-linoléoyl-2-O-butyl-1-α,β-D-désosamine | 52g<br>15g<br>32g<br>1g |

**Revendications**

1. Ethers et/ou esters de D-désosamine, caractérisés par le fait qu'ils répondent à la formule générale suivante:

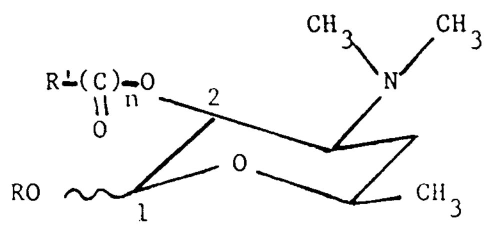

(I)

dans laquelle:

n représente 0 ou 1,

R et R′, identiques ou différents, représentent un radical alkyle, linéaire ou ramifié, ayant de 1 à 30 atomes de carbone, un radical alkényle ayant de 5 à 21 atomes de carbone, un radical cycloalkyle ayant de 4 à 10 atomes de carbone, un radical cycloalkylalkyle ayant de 5 à 11 atomes de carbone, un radical phényle ou arylalkyle éventuellement substitué(s), R pouvant en outre représenter un atome d'hydrogène quand n = 1 et R′ pouvant représenter un atome d'hydrogène quand n = O, le nombre des atomes de carbone de R, R′ ou R + R′ devant être compris entre 9 et 33 inclus,

ou R représente la partie aglyconique de l'érythralosamine et R′ représente dans ce cas un radical alkyle, linéaire ou ramifié ayant de 9 à 30 atomes de carbone, ou un radical alkényle ayant de 11 à 21 atomes de carbone,

et les anomères α et β et leurs mélanges ainsi que leurs sels.

**2.** Composés selon la revendication 1, caractérisés par le fait que le radical alkyle, linéaire ou ramifié, ayant de 1 à 30 atomes de carbone est le radical méthyle, éthyle, propyle, isopropyle, butyle, tert-butyle, octyle, nonyle, décyle, undécyle, dodécyle, tétradécyle, hexadécyle, octadécyle et décyl-2 tétradécyle.

**3.** Composés selon la revendication 1, caractérisés par le fait que le radical alkényle ayant de 5 à 21 atomes de carbone est le radical undécène-10-yle, heptadécaène-8-yle, heptadécadiène-8,11-yle et heptadécatriène-8,11,14-yle.

**4.** Composés selon la revendication 1, caractérisés par le fait que le radical cycloalkyle est le radical cyclopentyle, cyclohexyle ou adamantyle.

**5.** Composés selon la revendication 1, caractérisés par le fait que le radical phényle est substitué par au moins un atome de chlore, de brome, une fonction OH, un radical trifluorométhyle ou un radical alkyle inférieur.

**6.** Composés selon la revendication 1, caractérisés par le fait que le radical arylalkyle est le radical benzyle ou phénéthyle éventuellement substitué par au moins un atome de chlore, de brome, une fonction OH, un radical trifluorométhyle ou un radical alkyle inférieur.

**7.** Composés selon l'une quelconque des revendications 1 à 7, caractérisés par le fait qu'ils sont pris dans le groupe constitué par:

O-décanoyl-2-O-butyl-1-α,β-D-désosamine,
O-dodécanoyl-2-O-butyl-1-α,β-D-désosamine,
O-dodécanoyl-2-O-butyl-1-β-D-désosamine,
O-dodécanoyl-2-O-méthyl-1-α,β-D-désosamine,
O-dodécanoyl-2-O-méthyl-1-α-D-désosamine,
O-linoléoyl-2-O-butyl-1-α,β-D-désosamine,
O-linoléoyl-2-O-butyl-1-α-D-désosamine,
O-octadécyl-2-O-butyl-1-α,β-D-désosamine,
O-octadécyl-2-O-butyl-1-α-D-désosamine,
O-octadécyl-2-O-butyl-1-β-D-désosamine,
O-undécyl-2-O-décyl-1-α,β-D-désosamine,
O-undécyl-2-O-décyl-1-α-D-désosamine,
O-undécyl-2-O-décyl-1-β-D-désosamine,
di-O-octyl-1,2-α,β-D-désosamine,

di-O-octyl-1,2-α-D-désosamine,
di-O-octyl-1,2-β-D-désosamine,
O-(décyl-2)-tétradécyl-2-O-butyl-1,2-α,β-D-désosamine,
O-dodécanoyl-2′-érythralosamine,
O-oléoyl-2-α,β-D-désosamine,
O-décyl-1-α,β-D-désosamine,
O-décyl-1-α-D-désosamine,
O-décyl-1-β-D-désosamine,
O-dodécyl-1-α,β-D-désosamine,
O-hexadécyl-1-α,β-D-désosamine, et
O-(p-nonyl)phényl-1-α,β-D-désosamine.

**8.** Composés selon l'une quelconque des revendications 1 à 7, caractérisés par le fait qu'ils sont des esters-éthers respectivement en position 2 et en position 1 ou des diéthers, la somme des atomes de carbone de R + R′ étant comprise entre 12 et 30 inclus.

**9.** Procédé de préparation des composés de formule (I) sous forme d'esters, caractérisé par le fait qu'il consiste à faire réagir un excès d'un anhydride d'acide, préparé in situ, sur la D-désosamine ou un monoéther de D-désosamine en milieu solvant organique anhydre.

**10.** Procédé selon la revendication 9, caractérisé par le fait que le solvant organique est le tétrahydrofuranne, seul ou en mélange avec de la pyridine ou du N,N-diméthylformamide.

**11.** Procédé de préparation des composés de formule (I) sous forme d'éthers en position 1 de la D-désosamine, caractérisé par le fait qu'il consiste à faire réagir la D-désosamine sur un alcool ROH en présence d'un acide minéral ou organique, éventuellement dans un solvant organique.

**12.** Procédé selon la revendication 11, caractérisé par le fait que le solvant organique est le N,N-diméthylformamide.

**13.** Procédé de préparation des composés de formule (I) sous forme d'éthers en position 2 de la D-désosamine ($n = 0$), caractérisé par le fait qu'il consiste à faire réagir un halogénure R′X , X étant Cl ou Br, ou un dérivé tosylé sur un monoéther en 1 de la D-désosamine, en présence d'une base dans un solvant organique anhydre.

**14.** Procédé selon la revendication 13, caractérisé par le fait que la base est le tert-butanolate de potassium et le solvant organique, le dioxane ou le N,N-diméthylformamide.

**15.** Utilisation des composés selon l'une quelconque des revendications 1 à 8 comme agent anti-bactérien ou antifongique.

**16.** Composition pharmaceutique à usage humain ou vétérinaire, caractérisé par le fait qu'elle contient, dans un véhicule approprié, au moins un ester et/ou éther de D-désosamine tel que revendiqué selon l'une quelconque des revendications 1 à 8.

**17.** Composition cosmétique, caractérisée par le fait qu'elle contient dans un véhicule cosmétique approprié, au moins un ester et/ou éther de D-désosamine tel que revendiqué selon l'une quelconque des revendications 1 à 8.

**18.** Composition selon l'une quelconque des revendications 16 et 17, caractérisée par le fait qu'elle contient l'ester et/ou éther de D-désosamine en une concentration comprise entre 0,001 et 10% et de préférence entre 0,05 et 3% en poids par rapport au poids total de la composition.

## Claims

**1.** Ethers and/or esters of D-desosamine, characterized in that they correspond to the following general formula:

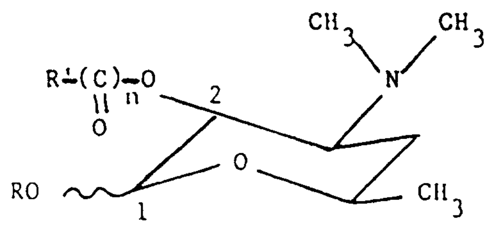

(I)

in which:

n represents 0 or 1,

R and R', which may be identical or different, represent a linear or branched alkyl radical having from 1 to 30 carbon atoms, an alkenyl radical having from 5 to 21 carbon atoms, a cycloalkyl radical having from 4 to 10 carbon atoms, a cycloalkylalkyl radical having from 5 to 11 carbon atoms, an optionally substituted arylalkyl or phenyl radical, it being additionally possible for R to represent a hydrogen atom when n = 1 and it being possible for R' to represent a hydrogen atom when n = 0, the number of carbon atoms of R, R' or R + R' having to be between 9 and 33 inclusively,

or R represents the aglycone portion of erythralosamine and R' represents, in this case, a linear or branched alkyl radical having from 9 to 30 carbon atoms or an alkenyl radical having from 11 to 21 carbon atoms, and the α and β anomers and their mixtures, as well as their salts.

**2.** Compounds according to Claim 1, characterized in that the linear or branched alkyl radical having from 1 to 30 carbon atoms is the methyl, ethyl, propyl, isopropyl, butyl, tert-butyl, octyl, nonyl, decyl, undecyl, dodecyl, tetradecyl, hexadecyl, octadecyl or 2-decyltetradecyl radical.

**3.** Compounds according to Claim 1, characterized in that the alkenyl radical having from 5 to 21 carbon atoms is the 10-undecenyl, 8-heptadecenyl, 8,11-heptadecadienyl or 8,11,14-heptadecatrienyl radical.

**4.** Compounds according to Claim 1, characterized in that the cycloalkyl radical is the cyclopentyl, cyclohexyl or adamantyl radical.

**5.** Compounds according to Claim 1, characterized in that the phenyl radical is substituted with at least one chlorine or bromine atom, an OH function, a trifluoromethyl radical or a lower alkyl radical.

**6.** Compounds according to Claim 1, characterized in that the arylalkyl radical is the benzyl or phenethyl radical optionally substituted with at least one chlorine or bromine atom, an OH function, a trifluoromethyl radical or a lower alkyl radical.

**7.** Compounds according to any one of Claims 1 to 7, characterized in that they are taken from the group consisting of:

2-O-decanoyl-1-O-butyl-α,β-D-desosamine,
2-O-dodecanoyl-1-O-butyl-α,β-D-desosamine,
2-O-dodecanoyl-1-O-butyl-β-D-desosamine,
2-O-dodecanoyl-1-O-methyl-α,β-D-desosamine,
2-O-dodecanoyl-1-O-methyl-α-D-desosamine,
2-O-linoleoyl-1-O-butyl-α,β-D-desosamine,
2-O-linoleoy1-1-O-butyl-α-D-desosamine,
2-O-octadecyl-1-O-butyl-α,β-D-desosamine,
2-O-octadecyl-1-O-butyl-α-D-desosamine,
2-O-octadecyl-1-O-butyl-β-D-desosamine,
2-O-undecyl-1-O-decyl-α,β-D-desosamine,
2-O-undecyl-1-O-decyl-α-D-desosamine,
2-O-undecyl-1-O-decyl-β-D-desosamine,
1,2-di-O-octyl-α,β-D-desosamine,
1,2-di-O-octyl-α-D-desosamine,
1,2-di-O-octyl-β-D-desosamine,
2-O-(2-decyl)tetradecyl-1-O-butyl-α,β-D-desosamine,

2'-O-dodecanoylerythralosamine,
2-O-oleoyl-α,β-D-desosamine,
1-O-decyl-α,β-D-desosamine,
1-O-decyl-α-D-desosamine,
1-O-decyl-β-D-desosamine,
1-O-dodecyl-α,β-D-desosamine,
1-O-hexadecyl-α,β-D-desosamine, and
1-O-(p-nonyl)phenyl-α,β-D-desosamine.

**8.** Compounds according to any one of Claims 1 to 7, characterized in that they are esters-ethers in position 2 and in position 1 respectively or diethers, the sum of the carbon atoms of R + R' being between 12 and 30 inclusively.

**9.** Process for the preparation of the compounds of formula (I) in the form of esters, characterized in that it consists in reacting an excess of an acid anhydride, prepared in situ, with D-desosamine or a monoether of D-desosamine in an anhydrous organic solvent medium.

**10.** Process according to Claim 9, characterized in that the organic solvent is tetrahydrofuran, alone or mixed with pyridine or N,N-dimethylformamide.

**11.** Process for the preparation of the compounds of formula (I) in the form of ethers in position 1 of D-desosamine, characterized in that it consists in reacting D-desosamine with an alcohol ROH in the presence of an inorganic or organic acid, optionally in an organic solvent.

**12.** Process according to Claim 11, characterized in that the organic solvent is N,N-dimethylformamide.

**13.** Process for the preparation of the compounds of formula (I) in the form of ethers in position 2 of D-desosamine ($n = 0$), characterized in that it consists in reacting a halide R'X, X being Cl or Br, or a tosyl derivative with a monoether in position 1 of D-desosamine, in the presence of a base in an anhydrous organic solvent.

**14.** Process according to Claim 13, characterized in that the base is potassium tert-butoxide and the organic solvent is dioxane or N,N-dimethylformamide.

**15.** Use of the compounds according to any one of Claims 1 to 8 as antibacterial or antifungal agents.

**16.** Pharmaceutical composition for human or veterinary use, characterized in that it contains, in a suitable vehicle, at least one ester and/or ether of D-desosamine as claimed according to any one of Claims 1 to 8.

**17.** Cosmetic composition, characterized in that it contains, in a suitable cosmetic vehicle, at least one ester and/or ether of D-desosamine as claimed according to any one of Claims 1 to 8.

**18.** Composition according to either of Claims 16 and 17, characterized in that it contains the ester and/or ether of D-desosamine at a concentration between 0.001 and 10%, and preferably between 0.05 and 3% by weight, relative to the total weight of the composition.

## Patentansprüche

1. Ether und/oder Ester von D-Desosamin, dadurch gekennzeichnet, daß sie der allgemeinen Formel

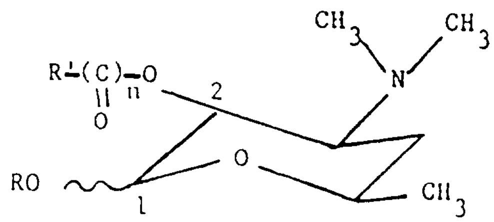

(I)

entsprechen, in der

n 0 oder 1 ist, und

R und R', die gleich oder verschieden sind, einen linearen oder verzweigten Alkylrest mit 1 bis 30 Kohlenstoffatomen, einen Alkenylrest mit 5 bis 21 Kohlenstoffatomen, einen Cycloalkylrest mit 4 bis 10 Kohlenstoffatomen, einen Cycloalkyllenrest mit 5 bis 11 Kohlenstoffatomen oder einen gegebenenfalls substituierten Phenyl- oder Aralkylrest darstellen, wobei R auch ein Wasserstoffatom bedeuten kann, wenn n = 1 ist, oder R' ein Wasserstoffatom sein kann, wenn n = 0 ist, und die Anzahl der Kohlenstoffatome der oben definierten Reste R, R' oder R + R' zwischen 9 und 33 einschließlich liegt,

oder in der R den aglykonischen Teil des Erythralosamins und R' in diesem Fall einen linearen oder verzweigten Alkylrest mit 9 bis 30 Kohlenstoffatomen oder einen Alkylenrest mit 11 bis 21 Kohlenstoffatomen darstellen,

sowie die α- und β-Anomere, deren Gemische wie auch deren Salze.

2. Verbindungen gemäß Anspruch 1, dadurch gekennzeichnet, daß der lineare oder verzweigte Alkylrest mit 1 bis 30 Kohlenstoffatomen eine Methyl-, Ethyl-, Propyl-, Isopropyl-, Butyl-, tert.-Butyl-, Octyl-, Nonyl-, Decyl-, Undecyl-, Dodecyl-, Tetradecyl-, Hexadecyl-, Octadecyl- oder Decyl-2-tetradecylgruppe ist.

3. Verbindungen gemäß Anspruch 1, daduch gekennzeichnet, daß der Alkylenrest mit 5 bis 21 Kohlenstoffatomen ein 10-Undecenyl-, 8-Heptadecenyl-, 8,11-Heptadecadienyl- oder 8,11,14-Heptadecatrienylrest ist.

4. Verbindungen gemäß Anspruch 1, dadurch gekennzeichnet, daß der Cycloalkylrest ein Cyclopentenyl-, Cyclohexyl- oder Adamantylrest ist.

5. Verbindungen gemäß Anspruch 1, dadurch gekennzeichnet, daß der Phenylrest mindestens mit einem Chlor- oder Bromatom, einer OH-Gruppe, einem Trifluormethyl- oder einem niedrigen Alkylrest substituiert ist.

6. Verbindungen gemäß Anspruch 1, dadurch gekennzeichnet, daß der Aralkylrest ein gegebenenfalls mit mindestens einem Chlor- oder Bromatom, einer OH-Gruppe oder einem Trifluormethyl- oder niedrigen Alkylrest substituierter Benzyl- oder Phenethylrest ist.

7. Verbindungen gemäß einem der Ansprüche 1 bis 6, dadurch gekennzeichnet, daß sie aus der folgenden Gruppe herausgegriffen sind:
O-Decanoy-2-O-butyl-1-α,β-D-desosamin,
O-Dodecanoyl-2-O-butyl-1-α,β-D-deosamin,
O-Dodecanoyl-2-O-butyl-β-D-desosamin,
O-Dodecanoyl-2-O-methyl-1-α,β-D-desosamin,
O-Dodecanoyl-2-O-methyl-1-α-D-desosamin,
O-Linoloyl-2-O-butyl-1-α,β-D-desosamin,
O-Linoloyl-2-O-butyl-1-α-D-desosamin,
O-Octadecyl-2-O-butyl-1-α,β-D-desosamin,
O-Octadecyl-2-O-butyl-1-α-D-desosamin,
O-Octadecyl-2-O-butyl-1-β-D-desosamin,

O-Undecyl-2-O-butyl-1-α,β-D-desosamin,
O-Undecyl-2-O-butyl-1-α-D-desosamin,
O-Undecyl-2-O-butyl-1-β-D-desosamin,
Di-O-octyl-1,2-α,β-D-desosamin,
Di-O-octyl-1,2-α-D-desosamin,
Di-O-octyl-1,2-β-D-desosamin,
O-(Decyl-2)-tetradecyl-2-O-butyl-1,2-α,β-D-desosamin,
O-Dodecanoyl-2'-erythralosamin,
O-Oleyl-2-α,β-D-desosamin,
O-Decyl-1-α,β-D-Desosamin,
O-Decyl-1-α-D-Desosamin,
O-Decyl-1-β-D-Desosamin,
O-Dodecyl-1-α,β-D-Desosamin,
O-Hexadecyl-1-α,β-D-Desosamin und
O-(p-Nonyl)-phenyl-1-α,β-D-Desosamin.

**8.** Verbindungen gemäß einem der Ansprüche 1 bis 7, dadurch gekennzeichnet, daß sie Ester bzw. Ether in den Positionen 1 und 2 oder Diether sind, wobei die Summe der Kohlenstoffatome von R + R' zwischen 12 und 30 einschließlich liegt.

**9.** Verfahren zur Herstellung von Verbindungen der Formel (I) in Form von Estern, dadurch gekennzeichnet, daß man einen Überschuß an in situ hergestelltem Säureanhydrid mit D-Desosamin oder einem D-Desosaminmonoether in einem Millieu aus einem wasserfreien organischen Lösungsmittel reagieren läßt.

**10.** Verfahren gemäß Anspruch 9, dadurch gekennzeichnet, daß das organische Lösungsmittel Tetrahydrofuran allein oder im Gemisch mit Pyridin oder N,N-Dimethylformamid ist.

**11.** Verfahren zur Herstellung von Verbindungen der Formel (I) in Form der Ether in Position 1 des D-Desosamins, dadurch gekennzeichnet, daß man das D-Desosamin mit einem Alkohol ROH in Anwesenheit einer organischen oder einer Mineralsäure, gegebenenfalls in einem organischen Lösungsmittel, reagieren läßt.

**12.** Verfahren gemäß Anspruch 11, dadurch gekennzeichnet, daß das organische Lösungsmittel N,N-Dimethylformamid ist.

**13.** Verfahren zur Herstellung von Verbindungen der Formel (I) in Form der Ether in Position 2 des D-Desosamins (n = 0), dadurch gekennzeichnet, daß man ein Halogenid R'X, wobei X Cl oder Br ist, oder ein Tosylderivat mit einem D-Desosamin-1-monoether in Anwesenheit einer Base in einem wasserfreien organischen Lösungsmittel reagieren läßt.

**14.** Verfahren gemäß Anspruch 13, dadurch gekennzeichnet, daß die Base Kalium-tert-butanolat und das organische Lösungsmittel Dioxan oder N,N-Dimethylformamid ist.

**15.** Verwendung der Verbindungen gemäß den Ansprüchen 1 bis 8 als Bakterizid oder Fungizid.

**16.** Pharmazeutische Zubereitung für die Verwendung am Mensch oder in der Veterinärmedizin, dadurch gekennzeichnet, daß sie in einem geeigneten Träger mindestens einen Ester und/oder Ether des D-Desosamins, gemäß einem der Ansprüche 1 bis 8 beansprucht, enthält.

**17.** Kosmetische Zubereitung, dadurch gekennzeichnet, daß sie in einem geeigneten kosmetischen Träger mindestens einen Ester und/oder Ether des D-Desosamins, gemäß einem der Ansprüche 1 bis 8, enthält.

**18.** Zubereitung gemäß einem der Ansprüche 16 und 17, dadurch gekennzeichnet, daß sie den D-Desosaminester und/oder -ether in einer Konzentration von 0,001 - 10 %, vorzugsweise von 0,05 - 3 Gew.- % enthält, bezogen auf das Gesamtgewicht der Zubereitung.